# EUROPÄISCHE PATENTSCHRIFT

(11) **EP 0 354 945 B1**
(45) Veröffentlichungstag und Bekanntmachung des Hinweises auf die Patenterteilung: **23.03.1994**
(21) Anmeldenummer: 89901715.6
(22) Anmeldetag: 11.01.1989
(51) Int. Cl.: C12N 9/00, C12N 1/38, C12N 1/00, C12N 5/04

(54) **VERFAHREN ZUR STEIGERUNG VON ENZYM-AKTIVITÄTEN UND DER SYNTHESELEISTUNG VON MIKROORGANISMEN UND HöHEREN PFLANZEN**
PROCESS FOR THE ENHANCEMENT OF ENZYME ACTIVITIES AND SYNTHETIC EFFICIENCY IN MICROORGANISMS AND HIGHER PLANTS
PROCEDE POUR AUGMENTER L'ACTIVITE ENZYMATIQUE ET LA CAPACITE BIOSYNTHETIQUE DANS DES MICROORGANISMES ET DES PLANTES SUPERIEURES

(30) Priorität: 13.01.1988 DE 3801023
(43) Veröffentlichungstag der Anmeldung: 21.02.1990
(73) Patentinhaber: SCHERING AKTIENGESELLSCHAFT, 13342 Berlin (DE)
(72) Erfinder: FIEDLER, Franz, D-8000 München 50 (DE); ZENK, Meinhart, H., D-8000 München 60 (DE); GUNDLACH, Heidrun, D-8046 Garching (DE); WEBER, Alfred, D-1000 Berlin 37 (DE); KENNECKE, Mario, D-1000 Berlin 33 (DE)
(86) Internationale Anmeldenummer: EP8900015
(87) Internationale Veröffentlichungsnummer: WO8906687

(56) Entgegenhaltungen:
- EP-A- 226 354
- Insect Biochem., Band 16, Nr. 13, 1986, Oxford, H. Yoshida et al: Microbial activation of two serine enzymes and prophenoloxidase in the plasma fraction of hemolymph of the silkworm, Bomyx Mori, Seiten 539-545
- Chemical Abstracts ,Band 99, Nr. 5, 1983 ( Columbus, Ohio, US W. F. Fett et al Bacterial growth and phytoalexin elicitation in soybean cell suspension cultures inoculated with Pseusomas syringae pathovars.

## Beschreibung

Die Erfindung betrifft ein Verfahren zur Steigerung von Enzym-Aktivitäten und der Syntheseleistung von Mikroorganismen und höheren Pflanzen, welches dadurch gekennzeichnet ist, daß man dieselben mit inaktivierten Elicitoren-haltigen Mirkoorganismen, Fragmenten derselben oder Exkretionen Elicitoren-haltiger Mikroorganismen in Kontakt bringt, mit der Maßgabe, daß man zur Steigerung von Enzym-Aktivitäten und der Syntheseleistung höherer Pflanzen inaktivierte Elicitoren-haltige Bakterien, Fragmente derselben oder Exkretionen Elicitoren-haltiger Bakterien verwendet.

Elicitoren sind bekanntlich mikrobielle oder pflanzliche Wirkstoffe, die mit Geweben höherer Pflanzen in Kontakt gebracht, deren Enzym-Aktivitäten und Syntheseleistung steigern. Die so in diesen Pflanzen akkumulierten Inhaltsstoffe werden, wenn sie antimikrobiell sind als Phytoalexine bezeichnet (Naturwissenschaften 68, 1981, 447ff, Adv. Enzymol, 55, 1983, 1ff und Spektrum der Wissenschaft 11, 1985, 85ff).

Gegenwärtig sind mehr als 100 Verbindungen, die der Phytoalexindefinition genügen, aus verschiedenen Pflanzenarten isoliert worden. Sie gehören verschiedenen Naturstoffgruppen an wie Terpenoiden, Linolensäurederivaten, Acetylenen und Polyacetylenen, Bibenzylen, Stilbenen, Phenanthrenen und Dihydrophenanthrenen, Benzofuranen und Phenolbenzofuranen, Furocumarinen, Avenaluminen, Flavanen, Phenylbenzofuranen, Benzoxazinonen, Alkaloiden, Isoflavonoiden (Brooks and Watson, Nat. Prod. Reports 2, 1985, 427).

Eine technische Anwendung hat diese Elicitoren-Wirkung bislang nicht gefunden. Dies hat mehrere Gründe: Es ist von wenigen Ausnahmen abgesehen bislang nicht möglich, Zellen höherer Pflanzen unter wirtschaftlich vertretbaren Bedingungen in Submerskulturen zu vermehren. Elicitoren in der Fermentation mittels Mikroorganismen anzuwenden, schien a priori zwecklos zu sein, da man nach der vorherrschenden Lehrmeinung über die Wirkungsweise von Elicitoren (Albersheim P. und Darvill A. G. Spektrum der Wissenhaft, 11, 1985, 85) davon ausgehen mußte, daß diese die Enzymaktivität und die Stoffwechselvorgänge in Mikroorganismen nicht beinflussen.

Bemerkenswert ist auch, daß nach geltender Lehrmeinung Bakterien eine Phytoalexinbildung bei Pflanzen nur auslösen können, indem sie, wie zum Beispiel Vertreter der Gattung Erwinia, mittels bestimmter Enzyme (Pektinasen) aus der pflanzlichen Zellwand Elicitoren (Oligogalakturonide) freisetzen, die als endogene Elicitoren die Phytoalexinbildung stimulieren.

Es wurde nun gefunden, daß Verbindungen und Zellpräparationen von Mikroorganismen, die im folgenden als Elicitoren bezeichnet werden, überraschenderweise doch in der Lage sind, in Mikroorganismen Enzymaktivitäten und deren Syntheseleistung zu steigern. Darüberhinaus wurde gefunden, daß es auch Bakterien gibt, die Elicitoren enthalten, welche keine Enzyme oder nutritive Faktoren sind.

Das erfindungsgemäße Verfahren kann grundsätzlich mittels isolierten oder synthetisierten Elicitoren durchgeführt werden, dies ist aber in der Regel viel zu aufwendig. Es genügt, wenn man inaktivierte Elicitoren-haltige Mikroorganismen verwendet, oder Fragmente dieser Mikroorganismen, wie zum Beispiel Zellwandfraktionen, Zellfragmente mechanisch aufgeschlossener oder chemisch bzw. enzymatisch lysierter Zellen oder durch Hilfsstoffe, wie zum Beispiel Ethanol oder Aceton präzipitierte Zellinhaltsstoffe. Inaktivierte Mikroorganismen sind im Sinne der Erfindung solche, die auf Dauer ihre Lebensfähigkeit verloren haben.

Wenn der Mikroorganismus Elicitoren in die Kulturbrühe abgibt, oder nach Lysis oder Sterilisation wasserlösliche Elicitoren-haltige Zellinhaltsstoffe bildet, so kann man auch diese Elicitoren-haltigen Exkretionen der Mikroorganismen zur Durchführung des erfindungsgemäßen Verfahrens verwenden. Jede Form Elicitoren-haltigen Produkts von Mikroorganismen eignet sich zur Durchführung des erfindungsgemäßen Verfahrens.

Mikroorganismen, von denen bekannt ist, daß sie Elicitoren besitzen sind unter anderem die in der nachfolgenden Tabelle 1 aufgelisteten Pilzstämme und Hefen:

**Tabelle 1**

| | |
|---|---|
| Alternaria carthami | Arch. Bioch.Biop, 229, 1984, 136 |
| Botrytis cinerea (ATCC 48345) | Physiol. Plant Pathol.11, 1977, 287 |
| Ceratocystis fimbriata | Phylochemistry 23, 1984,759 |
| Ceratocystis ulmi | Phytochemistry 23, 1984,383 |
| Chondrostereum purpureum | J.Chem.Soc.Perkin Trans I, 1984, 14341 |
| Cladosporium fulvum (ATCC 44961) | Physiol. Plant Pathol. 16, 1980, 391 |
| Colletotrichum lindemuthianum (ATCC 52471) | Eur. J. Biochem. 129, 1983,593 |
| Fusarium solani | Plant Physiol. 76, 1984,833 |
| Fusarium solanifspmori (ATCC 44934) | Nat.Prod. Rep. 2, 1985, 439 |
| Ganoderma applanatum | Phytochemistry 22, 1983,1039 |
| Glomerella cingulata | Physiol.Plant.Pathol. 21, 1982,171 |
| Helminthsporium carbonum (ATTC 52471) | Z. Naturforsch. Sect. C 38, 1983, 899 |
| Monilinia fructicola | J.Am.Chem. Soc.,84,1962,1919 |
| Nectria haematococca | Phytochemistry 22, 1983, 2291 |
| Phoma exigua | Phytochem. 21, 1982,1818 |
| Phytophthora cannabivora | Z.Naturf. Sect. C. 39, 1984,217 |
| Phytophthora capsici (ATCC 52771) | Physiol.Plat.Pathol. 18, 1981, 379 |
| Phytophthora infectans (ATCC 44776) | Phytochem.23, 1984,537 |
| Phytophthora megasperma var glycinea | Arch.Bioch. Bioph.229, 1984,136 |
| Phytophthora infectans | Phytpathol. Z. 27, 1956, 237 |
| Phytophthora megasperma | J. Biol. Chem. 259, 1984,11341 |
| Phytophthora nicotiane | Phytopathology 71, 1981, 864 |
| Puccinia coronata | Physiol. Plant Pathol. 20, 1982, 189 |
| Pyricularia oryzae (ATCC 15923) | Agric. Biol. Chem. 48, 1984, 253 |
| Saccharomyces cerevisiae | Plant Physiol. 62, 1978, 107 |
| Verticillium albo-atrum | Nat.Prod. Rep. 2, 1985,429 |
| Verticillium dahliae (ATCC 26289) | ATCC-Katalog. 16. Aufl. 1984 |

In eigenen Versuchen wurden proteolytisch mittels Trypsin gereinigte Zellwandpräparate von Gram-positiven Bakterienstämmen der Gattungen Bacillus, Corynebacterium, Brevibacterium, Cellulomonas, Lactobacillus, Pimelobacter, Rhodococcus und Staphylococcus und in Wasser hitzesterilisierte Mikroorganismen dieser Gattungen und deren Filtrate daraufhin untersucht, ob sie Elicitoren besitzen. In der nachfolgenden Tabelle 2 sind die Bakterienstämme aufgeführt, in denen Elicitoren nachgewiesen werden konnten.

**Tabelle 2**

| |
|---|
| Bacillus licheniformis ATCC 9945 |
| Bacillus pumilus ATCC 7061 |
| Brevibacterium butanicum ATCC 21196 |
| Brevibacterium flavum ATCC 13826, ATCC 14067 |
| Brevibacterium lactofermentum ATCC 13655 |
| Brevibacterium glutamingenes ATCC 13747 |
| Brevibacterium ammoniagenes ATCC 6872 |
| Corynebacterium hydrocarboclastum ATCC 15592 |
| Corynebacterium nephridii ATCC 11425 |
| Corynebacterium paurometabolum ATCC 8368 |
| Corynebacterium lilium ATCC 15990 |
| Corynebacterium striatum ATCC 6940 |
| Corynebacterium xerosis ATCC 373 |
| Corynebacterium diphtheriae (Stamm Mass. 8/Behring Werke) |
| Corynebacterium melassecola ATCC 17965 |
| Corynebacterium glutamicum ATCC 13032 |
| Corynebacterium uratoxidans ATCC 21749 |
| Lactobacillus casei subsp. rhamnosus ATCC 7469 |
| Lactobacillus plantarum DSM 20174 |
| Pimelobacter tumescens AJ 1460 |
| Rhodococcus fascians ATCC 12975 |
| Rhodococcus fascians 1 - Isolat von Prof. Dr. Stolp, Univ.Bayreuth |
| Rhodococcus fascians 2 - Isolat von Prof. Dr. Stolp, Univ.Bayreuth |

Im Rahmen der vorliegenden Erfindung wurden bislang nur Bakterienstämme weniger Gattungen von Gram-positiven Eubacterien daraufhin untersucht, ob sie Elicitoren besitzen. Auch unter den Pilzstämmen inklusive der Hefen wurden - soweit man den Vorpublikationen entnehmen kann - meist nur solche auf das Vorhandensein von Elicitor-Aktivitäten untersucht, von denen bekannt ist, daß sie phytopathogen sind. Es ist deshalb zu erwarten, daß zusätzlich noch zahlreiche Mikroorganismen, wie zum Beispiel Bakterien der Gattungen Mycobacterium, Nocardia, Nocardioides oder Pseudonocardia aufgefunden werden können, die ebenfalls Elicitoren besitzen.

Die Prüfung von Mikroorganismen auf Elicitor-Aktivität kann problemlos mittels der üblichen Screening-Versuche, wie sie dem Fachmann geläufig sind durchgeführt werden.

So kann man beispielsweise in Reihenversuchen die Mikroorganismen, deren Enzymaktivität oder Syntheseleistung gesteigert werden soll, in Submerskulturen anzüchten, den einzelnen Kulturen inaktivierte Mikroorganismen unterschiedlicher Spezies oder Subspezies zusetzen und nach erfolgter Fermentation analytisch ermitteln in welchen Kulturen eine Steigerung von Enzymaktivitäten oder der Syntheseleistung erzielt wird. Eine Steigerung von Enzymaktivitäten der Mikroorganismen erkennt man beispielsweise daran, daß man bei der fermentativen Umwandlung von Substraten eine erhöhte Bildungsgeschwindigkeit des - oder erhöhte Ausbeute an - Verfahrensprodukt erzielt. Entsprechend kann eine gesteigerte Syntheseleistung der Mikroorganismen beispielweise an einer erhöhten Bildungsgeschwindigkeit des - oder erhöhten Ausbeute an - Inhaltsstoffen des Mikroorganismus erkannt werden.

Wie die bislang durchgeführten Versuche, die in den Ausführungsbeispielen näher beschrieben werden, zeigen, scheint das erfindungsgemäße Verfahren zur Steigerung von Enzym-Aktivitäten oder der Syntheseleistung von Mikroorganismen sehr vielseitig anwendbar zu sein. So konnte durch Zugabe von Zellwandpräparationen von in der Tabelle 2 aufgeführten Mikroorganismen die Farbstoffbildung des Streptomyces lividans (Actinorhodin, Prodigiosin), sowie die Farbstoffbildung des Streptomyces coelicolor, des Streptomyces griseoruber, des Streptomyces latericius, des Streptomyces purpurascens und des Streptomyces violaceus stimuliert werden. Ferner war es möglich, die Bildung von β-Lactam-Antibiotika des Streptomyces clavuligerus und die Alkaloidsynthese von Claviceps paspali zu stimulieren.

Eine derartige Steigerung der Syntheseleistung von Mikroorganismen wird man nicht nur mittels der Zellpräparationen von den in der Tabelle 2 aufgeführten Bakterien erzielen können, sondern es ist zu erwarten, daß auch mittels Elicitoren-haligen Pilzen und Hefen, wie sie in der Tabelle 1 aufgeführt sind, eine Steigerung von Enzymaktivitäten und der Syntheseleistung von Mikroorganismen erzielt werden kann.

Es gelang ferner mit Hilfe von Zellwandpräparationen von in der Tabelle 2 aufgeführten Mikroorganismen in Zellkulturen höherer Pflanzen, wie Eschscholtzia californica oder Rauvolfia serpentina eine signifikante Steigerung der Alkaloidbildung zu erzielen.

Weiterhin gelang es mit Hilfe dieser Zellwandpräparationen, die Fähigkeit von Bacillus lentus, Steroide in der 1,2-Position zu dehydrieren, und die Fähigkeit von Rhodotorula glutinis, 17-Ketosteroide selektiv zu reduzieren, und die Fähigkeit von Penicillium raistrickii, Steroide in der 15α-Position zu hydroxylieren, deutlich zu steigern. Erfolglos blieb bislang lediglich der Versuch, mit Hilfe dieser Zellwandpräparation die Fähigkeit von Curvularia lunata zur 11β-Hydroxylierung von Steroiden zu steigern.

Diese Versuche lassen die Hoffnung berechtigt erscheinen, daß es mit Hilfe des erfindungsgemäßen Verfahrens möglich sein wird, auch die Bildung zahlreicher anderer gewerbsmäßig nutzbarer mikrobieller Inhaltsstoffe zu stimulieren und weitere Enzymaktivitäten von Mikroorganismen, die technisch nutzbar sind, zu steigern.

Derartige mikrobielle Inhaltsstoffe sind beispielsweise Antibiotika, wie die Penicilline, Cephalosporine, Cyclosporine, Actinomycine, Gramicidine, Neomycine, Gentamycine, Nystatine, Tetracycline, Nikomycine oder das Lincomycin, das Erythromycin, das Chloramphenicol, das Griseofulvin oder die Fusidinsäure u.a., Mutterkornalkaloide, wie das Ergocryptin, das Ergotamin, das Ergosin, das Ergocristin, das Ergocornin, das Agroclavin, das Chanoclavin, das Festuclavin, die Paspalinsäure oder die Lysergsäure-Derivate, Vitamine, wie das Vitamin B 12, das Riboflavin oder das β-Carotin, Enzyme wie die Amylasen, Glucoseisomerasen, Proteasen, Pectinasen, Cellulasen, Lipasen, Penicillinacylasen, Chitinase oder die Lactase, Nucleoside, wie die Guanylsäure oder Inosylsäure oder beispielsweise auch Aminosäuren, wie das Cystein, die Glutaminsäure, das Tryptophan, oder das Lysin.

Grundsätzlich sollte es auch möglich sein, bei genetisch veränderten Mikroorganismen, die endogene oder exogene Proteinbildung zu steigern. Solche nutzbaren Proteine sind nicht nur die bereits erwähnten Enzyme und Antibiotika, sondern auch beispielsweise Interferon, Insulin, Erythropoitin und TNF.

Mikroorganismen, die wegen ihrer Enzymaktivitäten technisch angewendet werden, sind beispielsweise in folgenden Publikationen beschrieben:
W. Charney and H. Herzog: Microbial Transformations of Steroids; Academic Press, New York etc. 1967
K. Kieslich: Microbial Transformations of Non-steroidal Cyclic Compounds; Georg Thieme Publ. Stuttgart (DE), 1976 und
K. Kieslich: Biotransformations; in H. J. Rehm und G. Reed (Herausgeber): Biotechnology; Weinheim (DE) etc. Vol 6a, 1984.
Derartige Mikroorganismen sind unter anderem solche, die Steroidtransformationen wie die 11α-, 11β-, oder 15α-Hydroxylierung, die Δ¹-Dehydrierung, 17α, 17β-Ketoreduktionen, oder den Seitenkettenabbau von Sterinen oder Antibiotikatransformationen, wie Penicillinspaltung bewirken.

Es ist nicht unwahrscheinlich, daß es mit Hilfe des erfindungsgemäßen Verfahrens gelingen könnte, neue technisch verwertbare mikrobielle Inhaltsstoffe, wie zum Beispiel neue Antibiotika aufzufinden, indem man den zu testenden Mikroorganismen inaktive Elicitoren-haltige Mikroorganismen zusetzt. Diese Hoffnung ist deshalb nicht unbegründet, weil bekannt ist, daß zahlreiche höhere Pflanzen Phytoalexine nur dann in nennenswerten Mengen bilden, wenn sie mit Elicitoren-haltigen Mikroorganismen infiziert sind.

Die Bestimmung, welcher Elicitoren-haltige Mikroorganismus die Enzymaktivitäten oder die Syntheseleistung eines bestimmten Mikroorganismus steigert, erfolgt mittels konventioneller Methoden, die dem Fachmann geläufig sind.

Die Durchführung des erfindungsgemäßen Verfahrens ist, soweit es die Fermentation mittels Mikroorganismen betrifft, für den Fachmann unproblematisch. Der Mikroorganismus, dessen Enzymaktivität oder dessen Syntheseleistung gesteigert werden soll, wird unter den bekannten Bedingungen angezüchtet; dann setzt man der Kultur die inaktivierten Elicitoren-haltigen Mikroorganismen, Fragmente derselben, Zellextrakte derselben oder Exkretionen derselben zu und setzt die Fermentation wie üblich fort. Die Zugabe der inaktivierten Mikroorganismen, der Fragmente oder Extrakte dieser höheren Pflanzen oder der Exkretionen von Elicitoren-haltiger Mikroorganismen kann bereits zu Fermentationsbeginn erfolgen. Die optimale Zugabezeit ist naturgemäß von der Art des angezüchteten Mikroorganismus abhängig, insbesondere vom Verlauf seiner exponentiellen Wachstumsphase und muß im Einzelfall ermittelt werden. So erweist es sich bei Bakterien beispielsweise oft als zweckmäßig, diese Zugabe 4 bis 30 Stunden nach Fermentationsbeginn durchzuführen. Bei der Zugabe inaktivierter Mikroorganismen oder Fragmente derselben verwendet man pro Kubikmeter Fermentationsbrühe üblicherweise 1 bis 1000 g (vorzugsweise 10 bis 200 g) inaktivierten Mikroorganismus oder 0,1 bis 100 g (vorzugsweise 1 bis 30 g) des Fragments dieses Organismus. Verwendet man Exkretionen von Elicitoren-haltigen Mikroorganismen so wird es in der Regel ausreichend sein, pro Kubikmeter Fermentationsvolumen 1 bis 50 l der Exkretions-Lösung anzuwenden. Wenn das erfindungsgemäße Verfahren dazu dient, die Enzymaktivität eines Mikroorganismus, der zur enzymatischen Umwandlung von Substraten angewendet wird, zu steigern, wird man die Zugabe des Substrates in der Regel 0 bis 10 Stunden nach erfolgter Zugabe des Elicitoren-haltigen inaktivierten Mikroorganismus oder dessen Fragmenten oder Exkretionen beginnen.

Die optimalen Fermentationsbedingungen sind von der Art des verwendeten Mikroorganismus, dem verwendeten Nährmedium, der Fermentationszeit, der Art und Menge des Elicitoren-haltigen Materials etc. abhängig; sie müssen im Einzelfall durch Vorversuche, wie sie dem Fachmann geläufig sind, ermittelt werden.

Zur Herstellung der inaktivierten Elicitoren-haltigen Mikroorganismen werden diese unter den üblichen Bedingungen angezüchtet, sodann durch Zentrifugieren oder Filtration von der Kulturbrühe getrennt, gewünschtenfalls gewaschen und nochmals isoliert. Zur Inaktivierung der Mikroorganismen können unterschiedliche Verfahren angewendet werden.

Mögliche Inaktivierungsmethoden bestehen darin, auf diese Mikroorganismen typische Zellgifte, wie Ethylenoxide, Formaldehyd, Ozon, Quecksilberverbindungen, organische Lösungsmittel, wie Methanol, Ethanol oder Aceton einwirken zu lassen oder die Mikroorganismen durch Erhitzen auf 90°C bis 140°C durch Einwirkung extremer Druckdifferenzen (Desintegration), Einwirken hochfrequenter elektrischer Felder oder durch UV-Bestrahlung, Bestrahlen mit γ-Strahlen oder Ultraschalleinwirkung abzutöten. Die Bedingungen, unter denen die Inaktivierung durchgeführt werden kann, sind dem Fachmann wohl bekannt. (K.H. Wallhäuser, H. Schmidt: Sterilisation, Desinfektion, Konservierung, Chemotherapie, Georg Thieme Verlag, Stuttgart (DE), 1967).

Fragmente Elicitoren-haltiger Mikroorganismen kann man beispielsweise durch Lysieren der Mikroorganismen durch Einwirken von osmotischen Schock oder Temperaturschock, durch Autolyse der Mikroorganismen, durch Behandeln der Zellen mit Ultraschall oder durch Zerreiben der Mikroorganismen mit Glasperlen, Glasstaub oder Quarzsand und anschließendem differenzierten Zentrifugieren erhalten (Hughes, D.E.,Wimpenny, J.W.T.and Lloyd, D.: The disintegration of micro-organisms. In: Methods in Microbiology, Vol13, (Norris, J. R. and Ribbons, D.W., eds.) pp. 1-54, Academic Press, New York, London, 1971).

Aus diesen Zellfragmenten können beispielsweise durch Trypsin-Behandlung gereinigte Zellwandfraktionen dargestellt werden. Die bereits erwähnten und in den nachfolgenden Ausführungsbeispielen verwendeten Zellwandfraktionen wurden nach dem von Schleifer und Kandler (Arch.Mikrobiol. 57, 1967, 335-365) beschriebenen Verfahren hergestellt.

Andererseits ist es aber auch möglich, aus wasserlöslichen Zellbestandteilen durch Ausfällen beispielsweise mit Ethanol oder Aceton Elicitoren-haltige Präzipitate herzustellen (Kocourek, J. and Ballou, C.E., J. Bacteriol. 100, 1969, 1175-1181).

Exkretionen Elicitoren-haltiger Mikroorganismen sind aktiv abgegebene, durch Lysieren, "leaky machen", Extraktion mit überkritischen verflüssigten Gasen (z.B. Kohlendioxid) oder Hitzesterilisation von Zellen in Wasser erhaltene Zellbestandteile, wasserlösliche oder durch Abfiltrieren oder Abzentrifugieren der Mikroorganismen und höheren Pflanzen erhaltene Kulturbrühen. Diese können bei Bedarf weiter aufgereinigt werden, beispielsweise durch Extraktion lipophiler Substanzen, Adsorption stark färbender Substanzen etc.

Es wurde bereits erwähnt, daß die Anwendung von enzymfreien Elicitorenhaltigem Material von Bakterien zur Steigerung der Leistung zur Synthese von Inhaltsstoffen höherer Pflanzen experimentell belegt werden konnte; dies könnte eventuell für die Anwendung pflanzlicher Zellkulturen zur Herstellung von Arzneimittelwirkstoffen von Bedeutung sein (M.H. Zenk in: Pharmazie heute, 103, 1982, Band 3, 131-138).

Die nachfolgenden Ausführungsbeispiele dienen zur näheren Erläuterung der Erfindung.

### Beispiel 1

Stimulation der Synthese von gefärbten Inhaltsstoffen (Actinorhodin, Prodigiosin) bei Streptomyces lividans (ATCC 19844) durch Zellwandpräparate.
80 ml eines Nährmediums bestehend aus
103 g Saccharose
10 g Glucose
10,12 g Magnesiumchlorid-Hexahydrat
0,25 g Kaliumsulfat
0,1 g Casaminoacids (Difco Labs, Detroit/USA)
800 ml destilliertes Wasser
werden sterilisiert (20 Minuten, 120° C) und mit folgenden frisch bereiteten Lösungen steril supplementiert.
1 ml 0,5 %iger Kaliumdihydrogen-Phosphat-Lösung
8 ml 3,68 %iger Calziumchlorid-Dihydrat-Lösung
1,5 ml 20 %iger L-Prolin-Lösung
10 ml 5,73 %iger TES-Puffer-Lösung (pH 7,2)
0,2 ml Spurenelementen-Lösung - enthaltend pro Liter
40 mg Zink(II)-chlorid
200 mg Eisen(III)-chlorid-Hexahydrat
10 mg Kupfer(II)-chlorid-Dihydrat
10 mg Mangan(II)-chlorid-Tetrahydrat
10 mg Dinatriumtetraborat-Dihydrat
10 mg Hexaammonium-heptamolybdat-Tetrahydrat
0,5 ml 1 N-Natronlauge
Je 1,8 ml dieser Nährlösung werden steril in die 24 jeweils 3 ml fassenden Kammern einer Polystyrol-Multischale (Multidish, Fa. Nunc, 6200 Wiesbaden 12) eingebracht. Je 2 mg der auf Elicitor-Gehalt zu testenden Zellwandpräparate werden in bidestilliertem Wasser 20 Minuten bei 120° C sterilisiert und die erhaltenen Suspensionen den Kammern zugesetzt. 2 Kammern erhalten keine Zusätze, sie dienen als Kontrollen. In allen Kammern wird das Volumen einheitlich mit bidestilliertem Wasser steril auf 2 ml eingestellt. Jede Kammer wird identisch mit 5 µl einer Sporensuspension von Streptomyces lividans (ATCC 19844) steril beimpft. Die Inkubation des Versuchsansatzes erfolgt aerob (Tablarschüttler; 100 Umdrehungen pro Minute) bei 26° C.

Nach 96 Stunden zentrifugiert man die Zellen ab, wäscht sie mit physiologischer Kochsalzlösung, trocknet sie im Vakuum über Kalziumchlorid und erhält so die in der Tabelle aufgeführten Zellausbeuten. Die bei der Zentrifugation erhaltenen Überstände werden auf einen pH-Wert von 7 eingestellt, auf 4 ml mit Wasser verdünnt und ihre Absorptionspektren zwischen 180 und 800 nm ermittelt. Die relativen Mengen der synthetisierten gelösten Sekundärstoffe Actinorhodin und Prodigiosin werden näherungweise durch Wiegen der Absorptionsgipfel der automatisch aufgezeichneten Spektren ermittelt.

Die nachfolgende Tabelle 3 zeigt die in dieser Versuchsreihe erzielten Ergebnisse.

**TABELLE 3**

| Getestete Bakterienzellwände von | Trockenzellausbeute (mg) | Farbstoffgehalt rel. Absorptionseinheiten |
|---|---|---|
| ohne (Kontrolle) | 22 | 1 |
| B. ammoniagenes (ATCC 6872) | 25 | 38 |
| B. glutamingenes (ATCC 13747) | 32 | 24 |
| Ba. pumilus (ATCC 7061) | 34 | 2 |
| B. linens (ATCC 19391) | 23 | 1 |
| C. diphtheriae (Mass. 8) | 24 | 34 |
| C. melassecola (ATCC 17965) | 26 | 34 |
| C. glutamicum (ATCC 13032) | 43 | 50 |
| C. lilium (ATCC 15990) | 33 | 40 |
| Ce. cellasea (ATCC 14359) | 36 | 2 |
| L. plantarum (DSM 20174) | 32 | 31 |
| S. aureus Stamm H | 44 | 1 |
| B = Brevibacterium Ba= Bacillus C = Corynebacterium Ce= Cellulomonas L = Lactobacillus S = Staphylococcus | | |

### Beispiel 2

Stimulation der Synthese von gefärbten Inhaltsstoffen (Actinorhodin, Prodigiosin) bei Streptomyces lividans (ATCC 19844) durch Zellwandextrakte.

Unter den Bedingungen des Beispiels 1, jedoch unter Verwendung der Sterilfiltrate der in bidestilliertem Wasser 20 Minuten bei 120° C sterilisierten 2 mg Zellwandpräparate wird eine fast gleich starke Stimulation der Farbstoffbildung bei Streptomyces lividans (ATCC 19844) erzielt, wie bei der Verwendung von Suspensionen dieser sterilisierten Zellwände.

### Beispiel 3

Stimulation der Synthese von gefärbten Inhaltsstoffen (Actinorhodin, Prodigiosin) bei Streptomyces lividans (ATCC 19844) durch Zellextrakte.

Unter den Bedingungen des Beispiels 2, jedoch unter Verwendung von je 20 mg Zellmasse anstelle von 2 mg Zellwandpräparat wird eine etwa gleich starke Stimulation der Farbstoffbildung erzielt, wie bei der Verwendung von Suspensionen der sterilisierten Zellwände.

### Beispiel 4

Stimulation der Synthese von gefärbten Inhaltsstoffen bei Streptomyces coelicolor A3(2) bzw. (ATCC 13 405).

Unter den Bedingungen des Beispiels 1, jedoch unter Verwendung von Streptomyces coelicolor A3(2) erzielt man eine deutliche Stimulation der Farbstoffbildung (wahrscheinlich ebenfalls Actinorhodin) bei diesem Bakterium.

### Beispiel 5

Stimulation der Synthese von gefärbten Inhaltsstoffen bei Streptomyces griseoruber (DSM 40275).

Unter den Bedingungen des Beispiels 1, jedoch unter Verwendung von Streptomyces griseoruber (DSM 40275) erzielt man bei diesem Bakterium ebenfalls eine sehr deutliche Steigerung der Farbstoffbildung (vermutlich Anthracyclin-Antibiotika).

### Beispiel 6

Stimulation der Synthese gefärbter Inhaltsstoffe bei Streptomyces purpurascens (DSM 40 310).

Unter den Bedingungen des Beispiels 1, jedoch unter Verwendung von Streptomyces purpurascens (DSM 40 310) erzielt man bei diesem Bakterium ebenfalls eine starke Steigerung der Farbstoffbildung (vermutlich ebenfalls Anthracyclin-Antibiotika).

### Beipiel 7

Stimulation der Synthese gefärbter Inhaltsstoffe bei Streptomyces latericius (DSM 40 163).

Unter den Bedingungen des beispiels 1, jedoch unter Verwendung von Streptomyces latericius (DSM 40 163) erzielt man bei diesem Bakterium ebenfalls eine signifikante Steigerung der Farbstoffbildung.

### Beispiel 8

Stimulation der Synthese gefärbter Inhaltsstoffe bei Streptomyces violaceus (DSM 40 082).

Unter den Bedingungen des Beispiels 1, jedoch unter Verwendung von Streptomyces violaceus (DSM 40 082) erzielt man bei diesem Bakterium ebenfalls eine signifikante Steigerung der Farbstoffbildung.

### Beispiel 9

Stimulation der Bildung von β-Lactam-Antibiotika (Cephalosporine, Penicillin N) durch Streptomyces clavuligerus (ATCC 27064).
5 g 3-(N-Morpholino)-propansulfonsäure (MOPS)
3,5 g Dikaliumhydrogenphosphat
0,6 g Magnesiumsulfat-Heptahydrat
2 g L-Asparagin
10 g Glycerin
1 g Hefeextrakt (Oxid, Wesel, DE)
1 ml Spurenelementsalz-Lösung - enthaltend pro Liter
1 g Eisen(II)-sulfat-Heptahydrat
1 g Mangan(II)-chlorid-Tetrahydrat
1 g Zinkchlorid-Heptahydrat
1 g Kalziumchlorid
werden mit destilliertem Wasser auf 1 Liter aufgefüllt und sterilisiert (20 Minuten; 120°C).

Je 1,8 ml dieser Nährlösung werden steril in 3 ml fassende Kammern einer sterilen Polystyrol-Multischale (Multidish; Fa. Nunc, 62 Wiesbaden 12) eingebracht. Je 2 mg der auf Elicitor-Aktivität zu testenden Zellwandpräparate oder 10 mg der zu testenden Zellen werden als in bidestilliertem Wasser sterilisierte (20 bzw. 45 Minuten 120° C) homogene Suspensionen zugesetzt. Zwei Kammern, die als Kontrolle dienen, erhalten keine Zusätze. In allen Kammern wird sodann das Volumen einheitlich mit bidestilliertem Wasser steril auf 2 ml eingestellt. Jede Kammer wird identisch mit 5 µl einer Sporensuspension von Streptomyces clavuligerus (ATCC 27064) beimpft.

Die Inkubation der Versuchsreihe wird unter aeroben Bedingungen (Tablarschüttler; 160 Umdrehungen pro Minute) bei 26° C vorgenommen. Die Inkubationszeit beträgt 24-48 Stunden.

Die Antibiotikumbildung wird im Vergleich mit den Kontrollen mit Hilfe des Plattendiffusionstests überprüft. Die in Soft-Nähragar suspendierten Detektororganismen sind Micrococcus luteus bzw. Bacillus subtilis (10 ⁶ Zellen pro ml). Auf Standardfilterplättchen (0,9 cm Durchmesser) werden jeweils 25 µl zentrifugierte (48.000 x g) Kulturbrühe aus den Testkammern appliziert. Nach einer Diffusionszeit von 4 Stunden bei 4° C wird der Biotest für 24 Stunden bei 30° C inkubiert.

Bei den Kulturen, die unter Zusatz abgetöteter Zellen von Brevibacterium flavum ATCC 13826, oder von Zellwandpräparationen dieses Bakteriums beziehungsweise von Zellwandpräparationen von Corynebacterium diphtheriae (Stamm Mass. 8) angezüchtet wurden, zeigten eindeutig vergrößerte Hemmhöfe im Vergleich zu den Kontrollen eine vermehrte Bildung von β-Lactam-Antibiotika (Penicillin N, Cephalosporine) durch Streptomyces clavuligerus an.

### Beispiel 10

Stimulation der Bildung von Alkaloiden (Sanguinarin, Chelirubin, Marcarpin und Chelerythrin) durch Kulturen von Eschscholtzia californica.

In 24 1 ml Kammern einer Polystrol-Multischale (Fa. Nunc, 6200 Wiesbaden 12) werden unter den von J. Berlin et. al. (Z. Naturforsch.,38c, 1983, 346-352) als optimal beschriebenen Bedingungen jeweils Gewebekulturen von Eschscholtzia californica angezüchtet. Eine der Kammern bleibt ohne weiteren Zusatz als Kontrolle, eine Kammer erhält 266 mg/l hitzeextrahierten und ethanolgefällten Hefeelicitor (hergestellt nach Kocourek J. and Ballou, C.E., J. Bacteriol 100, 1969, 1175-1181), die übrigen je 266 mg/l Zellwandpräparat der Bakterien, die in Tabelle 3 aufgeführt sind. Dann werden die Kulturen 72 Stunden lang bei 24°C inkubiert und anschließend der Alkaloidgehalt der Kulturen photometrisch ermittelt, wobei der durch den Hefeelicitor induzierte Alkaloidgehalt als 100 % gewertet wird.

Die nachfolgende Tabelle 4 zeigt die in dieser Versuchsreihe erzielten Ergebnisse.

**TABELLE 4**

| Getestete Bakterienzellwände | % Elicitoraktivität |
|---|---|
| Brevibacterium butanicum ATCC 21196 | 19 |
| Brevibacterium flavum ATCC 13826 | 16 |
| Brevibacterium flavum ATCC 14067 | 30 |
| Brevibacterium glutamingenes ATCC 137 | 113 |
| Brevibacterium lactofermentum ATCC 13655 | 43 |
| Brevibacterium ammoniagenes ATCC 6872 | 40 |
| Corynebacterium hydrocarboclastum ATCC 15592 | 8 |
| Corynebacterium nephridii ATCC 11425 | 123 |
| Corynebacterium paurometabolum ATCC 8368 | 16 |
| Corynebacterium lilium ATCC 15990 | 108 |
| Corynebacterium striatum ATCC 6940 | 17 |
| Corynebacterium petrophilum ATCC 19080 | 0 |
| Corynebacterium xerosis ATCC 373 | 102 |
| Corynebacterium diphtheriae Stamm Mass. 8 | 137 |
| Rhodococcus fasciens ATCC 12975 | 27 |
| Rhodococcus fascians 1 Isolat von Prof. Dr. Stolp,Univ.Bayreuth | 11 |
| Rhodococcus fascians 2 Isolat von Prof. Dr. Stolp,Univ.Bavreuth | 7 |

### Beispiel 11

Stimulierung der Bildung von Indolalkaloiden (Vallesiacotamin) in Kulturen von Rauvolfia serpentina.

Die Suspensionskultur von Rauvolfia serpentina (Stöckigt, J., A. Pfitzner and J. Firl: Plant Cell Rep. 1, 36-39 (1981) wird in Linsmaier und Skoog (LS)-Medium (Physiol. Plantarum 18, 100-127 (1965) auf Rotationsschüttlern (100 Umdrehungen pro Minute) bei 23° C und Dauerlicht (600 Lux) kultiviert. Zur Elicitierung werden 200 g Zellfrischgewicht/l LS-Medium angeimpft. Als Elicitoren-haltige Fragmente von Mikroorganismen werden Zellwandpräparate der in der Tabelle 4 aufgeführten Bakterien in einer Konzentration von 130 mg/l Medium verwendet.

Nach einer Inkubation von 5 Tagen hat sich sowohl in den elicitierten Kulturen als auch in den Kontrollen die Zellmasse verdoppelt. Die Zellen werden geerntet und mit Methanol extrahiert.

Die Menge des Indolalkaloids Vallesiacotamin wird über eine HPLC-Auftrennung der Extrakte bestimmt. Während die unbehandelten Kontrollkulturen nur 1,16 mg/l Medium enthalten, beträgt die Ausbeute bei den elicitierten Kulturen maximal 58 mg/l . Dies entspricht einer Steigerung um das 50-fache durch den Elicitor.

### Beispiel 12

Stimulierung der 17-Ketosteroid-Reduktase-Aktivität von Rhodotorula glutinis IFO 0389.
a) Ein 2 l Erlenmeyerkolben mit 500 ml sterilem Nährmedium enthaltend
   5 % Glucose-Monohydrat
   2 % Cornsteep liquor
   -eingestellt auf pH 6,5 -
   wird mit einem Abstrich aus einer Schrägagarkultur von Rhodotorula glutinis IFO 0389 beimpft und 40 Stunden lang bei 30° C mit 190 Umdrehungen pro Minute angezüchtet.
b) Ein 500 ml Erlenmeyerkolben mit 100 ml sterilen Nährmediums enthaltend
   1 % Cornsteep liquor
   5 % Nurupan^{(R)} (Hersteller Nurupan GmbH, 4000 Düsseldorf 1; DE)
   1 % Metarin^{(R)} (Hersteller Lucas Meyer; 2000-Hamburg 28; DE)
   - eingestellt auf pH 6,2 -
   wird mit 10 ml der gemäß Beispiel 12a hergestellten Rhodotorula-Vorkultur beimpft und 7 Stunden lang bei 30° C und 180 Umdrehungen pro Minute angezüchtet.
   Danach setzt man der Kultur 10 mg 3-Hydroxy-1,3,5(10),7-estratetraen-17-on zu und fermentiert weitere 210 Stunden lang. Dann extrahiert man die Kultur mit Methylisobutylketon, engt den Extrakt ein und reinigt das erhaltene Rohprodukt durch Chromatographie über eine Kieselgelsäule. Man erhält so 5,9 mg 1,3,5(10),7-Estratetraen-3,17α-diol = 59 % der Theorie.
c) Unter den Bedingungen des Beispiels 12b werden 10 mg 3-Hydroxy-1,3,5(10),7-estratetraen-17-on mit einer Kultur von Rhodotorula glutinis fermentiert, jedoch mit dem Unterschied, daß man dieser Kultur unmittelbar vor der Substratzugabe 5 ml einer sterilen Suspension von 50 mg einer Zellwandpräparation von Bacillus licheniformis (ATCC 9945) in Wasser zusetzt. Nach Aufarbeitung der Kultur erhält man 6,8 mg 1.3,5(10),7-Estratetraen-3,17-diol = 68 % der Theorie.

### Beispiel 13

Stimulierung der Steroid-Δ¹-Dehydrase-Aktivität von Bacillus lentus (ATCC 13805).
a) Ein 2 l Erlenmeyerkolben mit 500 ml steriler Nährlösung enthaltend
   0,5 % Cornsteep liquor
   0,05 % Glucose-Monohydrat
   0,1 % Hefeextrakt
   - eingestellt auf pH 7,0 -
   wird mit einer Abschwemmung von Bacillus lentus (ATCC 13 805) beimpft und 48 Stunden lang bei 30° C mit 190 Umdrehungen pro Minute geschüttelt.
b) Ein 500 ml Erlenmeyerkolben mit 100 ml steriler Nährlösung enthaltend
   3,0 % Sojapuder
   0,5 % Cornsteep liquor
   0,1 % Hefeextrakt
   0,05 % Glucose-Monohydrat
   -eingestellt auf pH 7,3 -
   wird mit 10 ml der Bacillus lentus Vorkultur beimpft und 7 Stunden lang bei 30° C mit 180 Umdrehungen pro Minute geschüttelt. Dann setzt man der Kultur eine sterilfiltrierte Lösung von 40 mg 6α,9α-Difluor-11β,17α-dihydroxy-16α-methyl-4-pregnen-3,20-dion in 4 ml Dimethylformamid zu und inkubiert weitere 41 Stunden lang.
   Dann extrahiert man die Kultur mit Methylisobutylketon, engt den Extrakt im Vakuum ein und reinigt den Rückstand durch Chromatographie über eine Kieselgelsäule. Man erhält so 16 mg 6α,9α-Difluor-11β,17α-dihydroxy-16α-methyl-1,4-pregnadien-3,20-dion (= 40 % der Theorie).
c) Unter den Bedingungen des Beispiels 13b werden 40 mg 6α,9α-Difluor-11β,17α-dihydroxy-16α-methyl-4-pregen-3,20-dion mit einer Kultur von Bacillus lentus fermentiert, jedoch mit dem Unterschied, daß man dieser Kultur unmittelbar vor der Substratzugabe 5 ml einer sterilen Suspension von 50 mg Zellwandpräparation von Corynebacterium diphtheriae (Stamm Mass. 8) in Wasser zusetzt. Nach Aufarbeitung der Kultur erhält man 21 mg 6α,9α-Difluor-11β,17α-dihydroxy-16α-methyl-1,4-pregnadien-3,20-dion (= 52,5 % der Theorie).

### Beispiel 14

Stimulierung der Bildung von Alkaloiden (Lysergsäureamid und Isolysergsäureamid) von Claviceps paspali (ATCC 13895).
a) Ein 500 ml Erlenmeyerkolben mit 50 ml einer sterilen Nährlösung enthaltend
   4 % Sorbit (technisch rein)
   1 % Glucose-Monohydrat
   2 % Bernsteinsäure
   0,6 % Ammoniumsulfat
   0,5 % Hefeextrakt (Difco^{(R)} der Difco Labs. Detroit/USA)
   0,1 % Kaliumdihydrogenphosphat,
   0,03% Magnesiumsulfat-Heptahydrat
   - eingestellt mit Natronlauge auf pH 5,2 -
   wird mit einer auf -70° C tiefgefrorenen Kultur von Claviceps paspali (ATCC 13895) beimpft und 5 Tage lang bei 24° C und 240 Umdrehungen pro Minute geschüttelt.
b) Ein 500 ml Erlenmeyerkolben mit 50 ml einer sterilen Nährlösung enthaltend
   8 % Sorbit (technisch rein)
   6 % Bernsteinsäure
   0,9 % Ammoniumsulfat
   0,1 % Calziumnitrat-Tetrahydrat
   0,05 % Dikaliumhydrogenphosphat
   0,03 % Magnesiumsulfat-Heptahydrat
   0,02 % Hefeextrakt (Difco^{(R)} der Difco Labs., Detroit/USA)
   0,0007 % Eisen(II)-sulfat-Heptahydrat
   0,0006 % Zinksulfat-Heptahydrat
   - eingestellt mit Natronlauge auf pH 5,2 -
   wird mit 5 ml Vorkultur von Claviceps paspali beimpft und 250 Stunden lang bei 24° C mit 240 Umdrehungen pro Minute geschüttelt.
   Dann setzt man der Kultur soviel Natronlauge zu, daß ein pH-Wert von mindestens 10 erreicht ist, extrahiert mit Methylisobutylketon, engt die Extrakte im Vakuum ein und reinigt sie durch Chromatographie über eine Kieselgelsäule.
   Man erhält so 35 mg eines Gemisches aus Lysergsäureamid und Isolysergsäureamid (Ausbeute 700 mg/l Kultur).
c) Unter den Bedingungen der Beispiele 14b, wird eine Kultur von Claviceps paspali angezüchtet, jedoch mit dem Unterschied, daß man der Kultur nach 72 Stunden 5 ml einer sterilen Suspension von 25 mg Zellwandpräparation von Lactobacillus casei subsp. rhamnosus (ATCC 7469) in Wasser zusetzt. Nach Aufarbeitung der Kultur erhält man 45 mg eines Gemisches aus Lysergsäureamid und Isolysergsäureamid (Ausbeute 900 mg/l Kultur).

### Beispiel 16

### Stimulierung der 15α-Hydroxylase-Aktivität von Penicillium raistrickii (ATCC 10490).

a) Ein 2 l-Erlenmeyerkolben mit 500 ml sterilem Nährmedium enthaltend
   3 % Glucose-Monohydrat
   1 % Cornsteep liquor
   0,2 % Natriumnitrat
   0,05 % Magnesiumsulfat-Heptahydrat
   0,05 % Kaliumchlorid
   0,002 % Eisen(II)-sulfat-Hexahydrat
   0,1 % Kaliumdihydrogenphosphat
   0,2 % Dikaliumhydrogenphosphat
   -eingestellt auf pH 6,0-
   wird mit einem Abstrich aus einer Schrägagarkultur von Penicillium raistrickii (ATCC 10490) beimpft und 48 Stunden lang bei 30° C mit 180 Umdrehungen pro Minute angezüchtet.
b) Ein 500 ml-Erlenmeyerkolben mit 100 ml sterilem Nährmedium enthaltend
   1 % Cornsteep liquor
   3 % Glucose-Monohydrat
   0,1 % Kaliumdihydrogenphosphat
   0,2 % Dikaliumhydrogenphosphat
   0,05 % Magnesiumsulfat-Heptahydrat
   -eingestellt auf pH 6,0-
   wird mit 10 ml der gemäß a) hergestellten Penicillium-Vorkultur beimpft.
   Danach setzt man der Kultur 300 mg 13-Ethyl-4-gonen-3,17-dion zu und fermentiert 120 Stunden bei 30° C mit 180 Umdrehungen pro Minute.
   Dann extrahiert man die Kultur mit Methylisobutylketon, engt den Extrakt ein und reinigt das erhaltene Rohprodukt durch Chromatographie über eine Kieselgelsäule. Man erhält so 180 mg 13-Ethyl-15α-hydroxy-4-gonen-3,17-dion.
c) Unter den Bedingungen von b) werden 300 mg 13-Ethyl-4-gonen-3,17-dion mit einer Kultur von Penicillium raistrickii fermentiert, jedoch mit dem Unterschied, daß man dieser Kultur unmittelbar vor der Substratzugabe 5 ml einer sterilen Suspension zugibt, die 50 mg einer Zellwandpräparation von Corynebacterium diphtheriae (Stamm Mass. 8) in Wasser enthält. Nach Aufarbeitung der Kultur erhält man 210 mg 13-Ethyl-15α-hydroxy-4-gonen-3,17-dion.

## Patentansprüche

1. Verfahren zur Steigerung von Enzym-Aktivitäten und der Syntheseleistung von Mikroorganismen und höheren Pflanzen, dadurch gekennzeichnet, daß man dieselben mit inaktivierten Elicitoren-haltigen Mikroorganismen, Fragmenten derselben oder Exkretionen Elicitoren-haltiger Mikroorganismen in Kontakt bringt, mit der Maßgabe, daß man zur Steigerung von Enzym-Aktivitäten und der Syntheseleistung höherer Pflanzen inaktivierte Elicitoren-haltige Bakterien, Fragmente derselben oder Exkretionen Elicitoren-haltiger Bakterien verwendet.

2. Verfahren zur Steigerung von Enzym-Aktivitäten und der Syntheseleistung von Mikroorganismen gemäß Patentanspruch 1, dadurch gekennzeichnet, daß man dieselben mit inaktivierten Elicitoren-haltigen Mikroorganismen, Fragmenten derselben oder Exkretionen Elicitoren-haltiger Mikroorganismen in Kontakt bringt.

3. Verfahren zur Steigerung der Syntheseleistung von Mikroorganismen gemäß Patentanspruch 2, dadurch gekennzeichnet, daß man zur Farbstoffbildung, zur Alkaloidbildung oder zur Antibiotikabildung befähigte Bakterien, Pilze und Hefen mit inaktivierten Elicitoren-haltigen Bakterien, Pilzen oder Hefen, Fragmenten derselben oder Exkretionen dieser Mikroorganismen in Kontakt bringt.

4. Verfahren zur Steigerung von Enzym-Aktivitäten von Mikroorganismen gemäß Patentanspruch 2, dadurch gekennzeichnet, daß man zur Steroidtransformation befähigte Bakterien, Pilze oder Hefen mit inaktivierten Elicitoren-haltigen Bakterien, Pilzen oder Hefen, Fragmenten derselben oder Exkretionen dieser Mikroorganismen in Kontakt bringt.

5. Verfahren zur Steigerung der Syntheseleistung von höheren Pflanzen gemäß Patentanspruch 1, dadurch gekennzeichnet, daß man Zellkulturen von zur Farbstoffsynthese, zur Alkaloidsynthese oder zur Phytoalexinsynthese befähigten höheren Pflanzen mit inaktivierten Elicitoren-haltigen Bakterien, Fragmenten derselben oder Exkretionen dieser Bakterien in Kontakt bringt.

6. Verfahren zur Steigerung von Enzym-Aktivitäten und der Syntheseleistung von Mikroorganismen und höheren Pflanzen gemäB Patentanspruch 1 bis 5, dadurch gekennzeichnet, daß man dieselben mit in Wasser hitzesterilisierten Elicitoren-haltiger Mikroorganismen, oder mit Filtraten derselben in Kontakt bringt.

## Claims

1. Method of enhancing enzyme activities and the synthetic capacity of microorganisms and higher plants, characterised in that the same are brought into contact with inactivated elicitor-containing microorganisms, fragments of the same or secretions of elicitor-containing microorganisms, with the proviso that, in order to enhance enzyme activities and the synthetic capacity of higher plants, inactivated elicitor-containing bacteria, fragments of the same or secretions of elicitor-containing bacteria are used.

2. Method of enhancing enzyme activities and the synthetic capacity of microorganisms according to patent claim 1, characterised in that the same are brought into contact with inactivated elicitor-containing microorganisms, fragments of the same or secretions of elicitor-containing microorganisms.

3. Method of enhancing the synthetic capacity of microorganisms according to patent claim 2, characterised in that bacteria, fungi and yeasts capable of forming dyestuffs, alkaloids or antibiotics are brought into contact with inactivated elicitor-containing bacteria, fungi or yeasts, fragments of the same or secretions of those microorganisms.

4. Method of enhancing enzyme activities of microorganisms according to patent claim 2, characterised in that bacteria, fungi or yeasts capable of steroid transformation are brought into contact with inactivated elicitor-containing bacteria, fungi or yeasts, fragments of the same or secretions of those microorganisms.

5. Method of enhancing the synthetic capacity of higher plants according to patent claim 1, characterised in that cell cultures of higher plants capable of dyestuff synthesis, alkaloid synthesis or phytoalexin synthesis are brought into contact with inactivated elicitor-containing bacteria, fragments of the same or secretions of those bacteria.

6. Method of enhancing enzyme activities and the synthetic capacity of microorganisms and higher plants according to patent claims 1 to 5, characterised in that the same are brought into contact with elicitor-containing microorganisms heat-sterilised in water, or with filtrates of the same.

## Revendications

1. Procédé pour accroître les activités enzymatiques et le pouvoir de biosynthèse de microorganismes et plantes supérieures, procédé caractérisé en ce que l'on met ceux-ci ou celles-ci en contact avec des microorganismes inactivés contenant des éliciteurs (activants), leurs fragments ou des excrétions de microorganismes contenant des éliciteurs, avec la condition d'employer, pour accroître les activités enzymatiques et le pouvoir de synthèse de plantes supérieures, des bactéries inactivées contenant des éliciteurs, des fragments de ces bactéries ou leurs excrétions contenant des éliciteurs.

2. Procédé pour accroître les activités enzymatiques et le pouvoir de biosynthèse de microorganismes selon la revendication 1, procédé caractérisé en ce qu'on les met en contact avec des microorganismes inactivés contenant des éliciteurs, des fragments de ces microorganismes ou des excrétions de microorganismes contenant des éliciteurs.

3. Procédé pour accroître le pouvoir de biosynthèse de microorganismes selon la revendication 2, procédé caractérisé en ce que l'on met des bactéries, mycètes ou levures aptes à la formation de colorants, d'alcaloïdes ou d'antibiotiques, en contact avec des bactéries, mycètes ou levures inactivés contenant des éliciteurs, ou bien avec des fragments de ceux-ci ou des excrétions de ces microorganismes.

4. Procédé pour accroître les activités enzymatiques de microorganismes selon la revendication 2, procédé caractérisé en ce que l'on met des bactéries, mycètes ou levures aptes à transformer des stéroïdes, en contact avec des bactéries, mycètes ou levures inactivés contenant des éliciteurs, ou bien avec des fragments de ceux-ci ou des excrétions de ces microorganismes.

5. Procédé pour accroître le pouvoir de synthèse de plantes supérieures selon la revendication 1, procédé caractérisé en ce que l'on met en contact des cultures cellulaires de plantes supérieures aptes à la synthèse de colorants, alcaloïdes ou phytoalexines, avec des bactéries inactivées contenant des éliciteurs, des fragments de ces bactéries ou leurs excrétions.

6. Procédé pour accroître les activités enzymatiques et le pouvoir de synthèse de microorganismes et de plantes supérieurs selon les revendication 1 à 5, procédé caractérisé en ce qu'on les met en contact avec des microorganismes contenant des éliciteurs qui ont été stérilisés à la chaleur dans de l'eau ou avec des filtrats de ceux-ci.
